# EUROPEAN PATENT SPECIFICATION

(11) **EP 3 999 036 B1**
(45) Date of publication and mention of the grant of the patent: **08.11.2023**
(21) Application number: 20746279.7
(22) Date of filing: 15.07.2020
(51) Int. Cl.: A61K 9/14, A61K 47/12, A61K 47/26, A61P 25/06, A61K 31/4045

(54) **ORODISPERSIBLE POWDER COMPOSITION COMPRISING A TRIPTAN**
ORODISPERGIERBARE PULVERZUSAMMENSETZUNG MIT EINEM TRIPTAN
COMPOSITION DE POUDRE ORODISPERSIBLE COMPRENANT UN TRIPTANE

(30) Priority: 19.07.2019 EP 19382611
(43) Date of publication of application: 25.05.2022
(73) Proprietor: BioPharma Synergies, S. L., 08028 Barcelona (ES)
(72) Inventor: AGELL PUIG, Francesc, 08028 Barcelona (ES)
(74) Representative: Araujo, Daniel
(86) International application number: PCT/IB2020/056638
(87) International publication number: WO 2021/014275

(56) References cited:
- WO-A1-02/41920
- WO-A1-2013/175511
- JP-A- 2012 025 676

## Description

### Technical Field

The present invention relates to pharmaceutical compositions comprising a triptan as active ingredient, which are dosed in the form of stick packs as orodispersible powders.

### Background art

Triptans are a family of tryptamine-based drugs used as abortive medication in the treatment of migraines and cluster headaches, which were first introduced in the 1990s.

Triptans play a significant role in the migraine pharmacotherapy. The first member of this class, sumatriptan, and the second-generation agents such as zolmitriptan, eletriptan, rizatriptan, almotriptan, frovatriptan and naratriptan, are selective agonists of serotonin 5-hydroxytryptamine 5-HT_{1B} and 5-HT_{1D} receptors. Three main key actions are responsible for the relief of migraine headache: Normalization of dilated intracranial arteries through enhanced vasoconstriction, peripheral neuronal inhibition, and inhibition of transmission through second order neurons of the trigeminocervical complex.

Patients with migraine and physicians consider that the rapid effect of the medication is a very important attribute in the acute therapy of migraine. Rapid administration of triptans improves the efficacy and treatment satisfaction by preventing progression in the migraine cycle.

Oral triptans, active ingredients developed specifically for the treatment of acute migraine, are much more effective if they are taken when the headache is still mild to moderate intensity rather than moderate to severe. Delaying the administration of the medication increases the possibility of an incomplete response to treatment, and an incomplete relief of headache correlates highly with early recurrence of moderate to severe headache.

Triptans are an appropriate first line therapy for patients with mild to severe migraine and are used for rescue therapy when nonspecific medications are ineffective.

The oral route of administration is the preferred administration route for patients with migraine. Among the formulations that exist orally, conventional tablets are less effective and tolerable when the patient suffers from vomiting and nausea, which are common symptoms during an acute attack of migraine.

Orodispersible formulations are an alternative for patients who prefer not to take conventional tablets, for patients who have nausea and vomiting during an acute attack of migraine, and for patients with dysphagia. For example, in Dowson et al., Zolmitriptan orally disintegrating tablet is effective in the acute treatment of migraine, Cephalalgia, 2002, 22 (2), 101-6, it is disclosed how zolmitriptan orodispersible tablets are preferred over conventional tablets by 70% of patients with migraine. In Dowson et al., Patients with migraine prefer zolmitriptan orally disintegrating tablet to sumatriptan conventional oral tablet, Int. J. Clin. Pract., 2003, 57(7), 573-6, it is disclosed that patients with migraine prefer zolmitriptan orodispersible tablets to sumatriptan conventional tablets because of the greater ease of administration of the medication (85.5%), as it is a more convenient formulation to be taken (86.1%), and which makes it possible to maintain a more active lifestyle (65.5%).

Of the 7 triptans that exist, only 2 triptans (rizatriptan and zolmitriptan) have orodispersible formulations in the market. In the other 5 triptans (sumatriptan, eletriptan, almotriptan, frovatriptan and naratriptan), for the oral route of administration only conventional tablets are available on the Spanish market.

A common problem associated with orodispersible formulations of triptans is the unpleasant bitter taste they present, as disclosed in WO-A-2010/0723353. The consequence thereof is that it is very difficult to mask and to obtain an orodispersible formulation with a good acceptance by the patient, without using complex masking techniques that are expensive and that do not delay the release of the active principle. This difficulty is supported, for example, by Sheshala et al., Formulation and Optimization of Orally Disintegrating Tablets of Sumatriptan Succinate, Chem. Pharm. Bull., 2011, 59(8), 920-928, which discloses that, if sumatriptan is incorporated directly into orodispersible tablets, the objective of masking the bitter taste that it presents will be definitely useless. Also, in Kaushik et al., Recent Patents and Patented Technology Platforms for Pharmaceutical Taste Masking, Recent Pat. Drug Deliv. Form., 2014, 8 (1), 37-45, it is disclosed that the strategy of incorporating sweeteners in very bitter and water-soluble active ingredients will not be useful to mask the bad taste they present.

In the state of the art, different approaches to mask the bitterness of triptans have been disclosed.

For example, in WO-A-02/41920 it is disclosed the blending of active ingredients, including sumatriptan, with cyclodextrins to mask the taste of the active ingredients.

In WO-A-2004/009085 it is disclosed an uncoated, taste-masked sumatriptan tablet comprising an intragranular portion that comprises granules of sumatriptan or a pharmaceutically acceptable salt thereof, and one or more diluents and/or binders present in a sufficient amount to cause a taste-masking of the sumatriptan or a pharmaceutically acceptable salt thereof; and an extragranular portion comprising one or more pharmaceutically acceptable excipients around the intragranular granules; it further discloses a polishing process of sumatriptan tablets by spraying a solution or suspension of a wax component onto the tablets and/or dusting a wax powder onto the tablets.

In WO-A-2006/035313 it is disclosed an uncoated tablet of sumatriptan or a pharmaceutically acceptable salt thereof, comprising an intragranular portion that contains sumatriptan or a pharmaceutically acceptable salt thereof, and optionally a disintegrant and/or a surfactant, and an extragranular portion comprising a diluent, an alkalizing agent, a disintegrant and a lubricant. However, nothing is said regarding the acceptance of the tablets by patients.

In EP-A-1025858 it is described a composition that includes an active ingredient having an unpleasant taste and polyvinylpyrrolidone and/or copolyvidone. The composition may be solid, liquid, or semisolid. The unpleasant taste of the active ingredient is reportedly alleviated by adding polyvinylpyrrolidone and/or copolyvidone in amounts of 5 to 200 parts by weight per 1 part by weight of the active ingredient.

In WO-A-2007/130773 it is disclosed a rapidly disintegrating oral triptan composition, which comprises a triptan compound, a resin, a lubricant, a disintegrant, and a compressible material, where the triptan is admixed with the resin forming a taste-masked triptan composition, which is further admixed with the lubricant, the disintegrant, and the compressible material to form the rapidly disintegrating oral triptan composition.

In EP-A-2387993 it is disclosed a silicon dioxide free orally disintegrating tablet formulation of zolmitriptan or a pharmaceutically acceptable salt thereof, having heavy magnesium carbonate and sodium stearyl fumarate with one or more pharmaceutically acceptable excipients, which have a pleasant mouth feel.

In WO-A-2013/058496 it is disclosed a pharmaceutical composition for oral administration, wherein taste of the pharmaceutically active ingredient (e.g. sumatriptan) is masked, prepared by wet granulation of a mixture of a pharmaceutically active ingredient, and at least one compound selected from the group consisting of magnesium aluminometasilicate and calcium silicate, and a pharmaceutically acceptable carrier. The oral pharmaceutical composition may be prepared without using a costly special manufacturing system or additive, having a taste masking effect on active ingredients, and exhibiting an appropriate drug dissolution rate in the body in order to have a therapeutic effect.

In WO-A-2010/072353 it is disclosed an aqueous liquid pharmaceutical composition comprising a) at least one triptan compound, or a pharmaceutically acceptable salt or hydrate thereof, and b) xylitol.

In WO-A-2007/070504 it is disclosed an improved oral liquid composition that includes sumatriptan, or a pharmaceutically acceptable salt thereof, and a pharmaceutically acceptable carrier. Typically, the compositions include a sweetening agent and a flavouring agent, or a bitterness-reducing agent and a flavouring agent. It is further disclosed that glycerine and/or propylene glycol should be included in the formulation to provide sweetness and/or to reduce the bitterness of the drug.

Coating of the tablets is a common solution to mask the bitter taste, as taught by WO-A-01/37816, which discloses a process for the coating of sumatriptan tablets to reportedly provide a taste masking of the sumatriptan. The process includes spraying a coating solution or suspension of a sugar, a starch, or a mixture of a sugar and a starch, onto tablet cores to obtain coated tablets. A similar approach is followed by WO-A-2005/70417, which discloses a taste masking pharmaceutical composition for oral administration, comprising a core of active ingredient and one or more outer non-active taste masking layers formed on the core by application of pressure. In GB-A-2010/06489 it is disclosed a process for the preparation of an oral tablet comprising naratriptan, anhydrous lactose, a first microcrystalline cellulose, a second microcrystalline cellulose, croscarmellose sodium and magnesium stearate, wherein the process comprises the preparation of core tablets using the steps defined therein and coating the core tablets.

Though the coating may mask the unpleasant taste, if the thickness and composition of the coating is not properly controlled, it may affect the disintegration and dissolution characteristics of the tablet. Further, the coating operation is a highly controlled and costly process.

Despite the various proposals available in the state of the art, there is still a need to have new oral pharmaceutical compositions, comprising a triptan as active ingredient, having a masked taste and a high degree of acceptance by the patient.

### Object of the invention

The object of the present invention is an orodispersible powder composition.

A stick pack comprising the orodispersible powder composition is also part of the invention.

The orodispersible powder composition for use in the treatment of migraine is also part of the invention.

### Description of the Drawings

Figure 1 represents a mixture diagram to test ten specific combinations of sodium citrate, citric acid and sucralose, to obtain an equation to describe the behaviour of the ternary system. In that diagram, A represents sodium citrate, B citric acid and C sucralose.
Figure 2 represents the isolines corresponding to the degree of acceptance for the orodispersible powders containing sumatriptan as active pharmaceutical ingredient, and the synergistic ternary combination of sodium citrate (A), citric acid (B) and sucralose (C).
Figure 3 represents the isolines corresponding to the degree of acceptance for the orodispersible powders containing zolmitriptan as active pharmaceutical ingredient, and the synergistic ternary combination of sodium citrate (A), citric acid (B) and sucralose (C).

### Detailed description of the invention

The object of the present invention is an orodispersible powder composition, which comprises:
a) a pharmacologically effective amount of a triptan or a pharmaceutically acceptable salt thereof,
b) a ternary combination consisting of sodium citrate, citric acid and sucralose, and
c) one or more further excipients,

wherein the ratio a):b) is comprised from 1:2 to 1:90,
wherein the content of sodium citrate is comprised from 55% to 75% by weight over the total weight of the ternary combination,
wherein the content of citric acid is comprised from 15% to 35% by weight over the total weight of the ternary combination, and
wherein the content of sucralose is comprised from 10% to 30% by weight over the total weight of the ternary combination.

The authors of the present invention have developed an orodispersible powder by direct mixing with optimum flow properties, masking the bitter taste of the triptans, and exhibiting an excellent acceptance of the formulation by the patient. The masking of the bitter taste has been surprisingly produced by a synergistic effect between citric acid, sodium citrate and sucralose. The orodispersible formulation dissolves rapidly in the oral cavity (less than 15 seconds), leaving no product residue in the mouth, and without the need to drink water after administration. Additionally, orodispersible powders according to claim 1 show surprisingly optimal flow properties even after long periods of storage at room temperature.

The orodispersible powder according to claim 1 allows improving the treatment compliance in patients with migraine and reducing manufacturing costs. Further, the formulation is suitable for diabetics and for lactose intolerant.

In the context of the present invention, an orodispersible powder is a pharmaceutical composition, wherein the active pharmaceutical ingredient and the excipients are in powder form and which disperses and/or dissolves rapidly in the oral cavity. Rapidly means in less than 30 seconds, preferably in less than 20 seconds, and more preferably in less than 15 seconds.

In the context of the present invention, the term "triptan" refers both to triptan and to a pharmaceutically acceptable salt thereof.

In the present description, as well as in the claims, the singular forms "a", "an" and "the" include the plural reference unless the context clearly indicates otherwise. The ranges defined by the preposition "between" or by the terms "from ...to..." include also the two ends thereof. The term "about" refers to a deviation of plus/minus 10%, preferably plus/minus 5%. The percentages are expressed in % by weight, unless stated the contrary.

### Triptan

The active ingredient of the orodispersible powder of the present invention is a triptan or a pharmaceutically acceptable salt thereof.

As used herein, the term "pharmaceutically acceptable salt" refers to a salt prepared from pharmacologically acceptable anions, such as, for example, acetate, adipate, anthranilate, ascorbate, benzenesulfonate, benzoate, bisulfate, bitartrate, camphorsulfonate, ethanesulfonate, formate, fumarate, furoate, galacturonate, gentisate, gluconate, glucuronate, glutamate, glycolate, hydrobromide, hydrochloride, hydroiodide, isethionate, isonicotinate, lactate, malate, maleate, malonate, mandelate, mesylate, mucate, nitrate, pamoate, pantothenate, phentylacetate, phosphate, propionate, saccharate, salicylate, stearate, succinate, sulfanilate, sulfate, tartrate, p-toluenesulfonate, and hydrates thereof. Preferably the pharmaceutically acceptable salt is selected from benzoate, hydrobromide, hydrochloride, malate, succinate, and hydrates thereof.

Triptans are selective agonists of serotonin 5-hydroxytryptamine 5-HT_{1B} and 5-HT_{1D} receptors, which are used in the treatment of migraine.

Triptans are preferably selected from the group of sumatriptan, zolmitriptan, eletriptan, rizatriptan, almotriptan, frovatriptan, naratriptan, donitriptan, avitriptan, LY-334370, L-703,664 or GR 46611, and more preferably from sumatriptan, zolmitriptan, eletriptan, rizatriptan, almotriptan, frovatriptan, and naratriptan.

The pharmacologically effective amount of each triptan is well-known by the skilled person in the art. In commercially available medicinal products recommended triptan doses are, for example: 2.5 mg naratriptan, 2.5 mg frovatriptan, 5 mg zolmitriptan, 10 mg rizatriptan, 12.5 mg almotriptan, 40 mg eletriptan and 100 mg sumatriptan.

Triptans and pharmaceutically acceptable salts thereof are available active pharmaceutical ingredients, either commercially, for example donitriptan (Sigma Aldrich), avitriptan (Sigma Aldrich), LY-334370 (Sigma Aldrich), L-703,664 (Biogen), or GR 46611 (Sigma Aldrich), or they can be prepared according to processes already disclosed in prior art, for example, US4816470 (sumatriptan), US5466699 (zolmitriptan), WO-A-92/06973 (eletriptan), EP-A-0497512 (rizatriptan), WO-A-94/02460 (almotriptan), WO-A-93/00086 (frovatriptan), or EP-A-0303507 (naratriptan).

The content of triptan in the orodispersible powder of the invention is usually comprised from 0.05% to 10%, preferably from 0.1% to 5% by weight over the total weight of the orodispersible powder composition. If a pharmaceutically acceptable salt of a triptan is used in the formulation, then the amount is corrected accordingly.

### Ternary combination

The orodispersible powder of the invention comprises a synergistic ternary combination consisting of sodium citrate, citric acid and sucralose, preferably a ternary combination of sodium citrate anhydrous, citric acid anhydrous and sucralose, according to claim 1.

The synergistic ternary combination is formulated in the orodispersible powder of the invention in a ratio triptan ternary combination comprised between 1:2 and 1:90, preferably between 1:3 to 1:85 and more preferably between 1:5 and 1:80. In a preferred embodiment the ratio is selected from the group comprising: 1:2, 1:3, 1:4, 1:5, 1:6.5, 1:10, 1:15, 1:16, 1:20, 1:25, 1:30, 1:32, 1:35, 1:40, 1:45, 1:50, 1:55, 1:60, 1:65, 1:70, 1:75, 1:79, 1:80, 1:85, and 1:90; in a more preferred embodiment the ratio is selected from the group consisting of about 1:6.45, about 1:16.4, about 1:32, about 1:40 and about 1:79.

Sodium citrate, as either the dihydrate or anhydrous material, is used in pharmaceutical formulations. It is used primarily as a buffering agent to adjust the pH.

Citric acid as either the monohydrate or anhydrous material, is used in pharmaceutical formulations. It is used primarily as a buffering agent to adjust the pH.

In the composition of the invention, sodium citrate is preferably anhydrous and citric acid is preferably anhydrous.

Sucralose is used as a sweetening agent in pharmaceutical applications. It has a sweetening power approximately 300-1000 times that of sucrose and has no aftertaste. It has no nutritional value, is noncariogenic, does not promote dental caries, and produces no glycemic response.

In the ternary combination, the content of sodium citrate is comprised from 55% to 75%, and preferably from 60% to 70% by weight over the total weight of the ternary combination.

In the ternary combination, the content of citric acid is comprised from 15% to 35%, and preferably from 20% to 30% by weight over the total weight of the ternary combination.

In the ternary combination, the content of sucralose is comprised from 10% to 30%, and preferably from 15% to 25% by weight over the total weight of the ternary combination.

The amounts of sodium citrate, citric acid and sucralose in the ternary combination are combined to make 100%. If hydrated forms of sodium citrate and/or citric acid are used, the amounts are corrected accordingly.

The ternary combination comprises 55% to 75% by weight of sodium citrate, 15% to 35% by weight of citric acid and 10% to 30% by weight of sucralose, preferably 58% to 70% by weight of sodium citrate, 18% to 30% by weight of citric acid and 13% to 25% by weight of sucralose, and more preferably 60% to 65% by weight of sodium citrate, 20% to 25% by weight of citric acid and 15% to 20% by weight of sucralose, wherein the amounts of sodium citrate, citric acid and sucralose in the ternary combination are combined to make 100%.

The content of the ternary combination in the orodispersible powder composition is comprised between 9% and 50%, preferably between 9% and 45%, more preferably between 9% and 40%, more preferably between 9% and 35%, and yet more preferably between 9% and 30% by weight over the total weight of the orodispersible powder composition.

Surprisingly, the ternary combination is able to mask the bitter taste of triptans a long a broad range of ratios between triptan and ternary combination, 1:2 to 1:90, preferably 1:3 to 1:85, and more preferably 1:5 to1:80. As shown in the examples, a ratio triptan: ternary combination of about 1 :6.45 was exemplified for sumatriptan, about 1 :16.37 for eletriptan, about 1:31.68 for almotriptan, about 1:39.6 for rizatriptan, and about 1:79.2 for zolmitriptan, which is also suitable for naratriptan and frovatriptan.

### Other excipients

The orodispersible powder of the invention may comprise generally further excipients, such as diluents, lubricants, glidants, flavouring agents, sweetening agents, acidulants, alkalizing agents, buffering agents, antioxidants and mixtures thereof.

Suitable excipients for use in the preparation of the orodispersible powder of the invention are described, for example, in the book Handbook of pharmaceutical excipients, R.C. Rowe, P.J. Sheskey and M.E. Quinn, editors, Pharmaceutical Press, sixth edition, 2009.

Further excipients that are usually included in the orodispersible powder of the invention are selected from the group comprising diluents, lubricants, glidants, flavouring agents and mixtures thereof.

In a preferred embodiment, the orodispersible powder comprise one or more further excipients selected from diluents, lubricants, glidants, flavouring agents and mixtures thereof.

Suitable diluents may comprise, for example, mannitol, sorbitol, xylitol, isomalt, erythritol, cellulose powdered, microcrystalline cellulose, silified microcrystalline cellulose, starch, calcium phosphate, calcium lactate, calcium carbonate, magnesium carbonate, magnesium oxide and mixtures thereof; preferably the diluent is mannitol. In a preferred embodiment, the composition does not comprise lactose.

Mannitol is used in pharmaceutical formulations. It is primarily used as a diluent, where it is of particular value since it is not hygroscopic and may thus be used with moisture-sensitive active ingredients. It is commonly used as an excipient in the manufacture of chewable tablet formulations because of its negative heat of solution, sweetness, and 'mouth feel', imparting a cooling sensation in the mouth. It is also used as a diluent in rapidly dispersing oral dosage forms. Given orally, mannitol is not absorbed significantly from the gastrointestinal tract, and it is suitable for diabetic patients. Mannitol does not undergo Maillard reactions. It has a low caloric content and it is noncariogenic.

Generally, the diluent is present in an amount from 45% to 95%, preferably from 50% to 90%, more preferably from 55% to 85%, and yet more preferably from 60% to 85% by weight over the total weight of the orodispersible powder composition.

The orodispersible powder may further comprise a lubricant, wherein the lubricant is usually selected from the group consisting of talc, sodium benzoate, sodium stearyl fumarate, sodium lauryl sulfate, calcium stearate, magnesium stearate, zinc stearate, glyceryl behenate, stearic acid, glyceryl monostearate, poloxamer, polyethylene glycol and mixtures thereof; preferably the lubricant is sodium stearyl fumarate.

Sodium stearyl fumarate is used as a lubricant in pharmaceutical compositions. It is supplied in a pure form and is of value when the less pure stearate-type lubricants are unsuitable owing to chemical incompatibility. Sodium stearyl fumarate is less hydrophobic than magnesium stearate or stearic acid and has a less retardant effect on dissolution than magnesium stearate.

Generally, the lubricant is present in an amount from 0.05% to 10%, preferably from 0.1% to 5%, more preferably from 0.2% to 2.5%, more preferably from 0.4% to 1%, more preferably from 0.45% to 0.75%, and yet more preferably about 0.5% by weight over the total weight of the orodispersible powder composition.

The orodispersible powder may further comprise a glidant, wherein the glidant is usually selected from the group consisting of colloidal silicon dioxide, calcium phosphate tribasic, hydrophobic colloidal silica, magnesium silicate, magnesium trisilicate, silicon dioxide, talc and mixtures thereof. The preferred glidant is colloidal silicon dioxide.

Colloidal silicon dioxide is used in pharmaceutical formulations. Its small particle size and large specific surface area give it desirable flow characteristics that are exploited to improve the flow properties of dry powders.

Generally, the glidant is present in an amount from 0.05% to 10%, preferably from 0.1% to 5%, more preferably from 0.2% to 2.5%, more preferably from 0.4% to 1%, more preferably from 0.45% to 0.75%, and yet more preferably about 0.5% by weight over the total weight of the orodispersible powder composition.

The orodispersible powder may further comprise a flavouring agent, wherein it is usually selected from fruit flavours such as orange, banana, strawberry, cherry, wild cherry, lemon and mixtures thereof, and other flavours such as cardamom, anise, peppermint, menthol, vanillin and ethyl vanillin and mixtures thereof. preferably the flavouring agent comprises peppermint flavour.

Generally, the content of the flavouring agent is comprised from 0.05% to 5.0% by weight over the total weight of the orodispersible powder composition.

In some embodiments, the orodispersible powder may comprise a sweetening agent, which is usually selected from the group consisting of saccharin sodium, neohesperidin dihydrochalcone, acesulfame potassium, aspartame and mixtures thereof. Preferably the composition does not include sucrose, and therefore it is suitable for diabetics.

Generally, the sweetening agent is usually present in an amount from 0.1% to 5% by weight over the total weight of the orodispersible powder composition.

In some embodiments, the orodispersible powder may comprise an acidulant, which is usually selected from the group consisting of fumaric acid, maleic acid, malic acid, tartaric acid and mixtures thereof.

Generally, the content of the acidulant is comprised from 0.1% to 2.0% by weight over the total weight of the orodispersible powder composition.

In some embodiments, the orodispersible powder may comprise an alkalizing agent, which is usually selected from the group consisting of potassium bicarbonate, potassium citrate, sodium bicarbonate, sodium carbonate and mixtures thereof. Generally, the alkalizing agent is usually present in an amount from 1% to 40% by weight over the total weight of the orodispersible powder composition.

In some embodiments, the orodispersible powder may comprise a buffering agent, which is usually selected from the group consisting of dibasic sodium phosphate, glycine, methionine and mixtures thereof.

Generally, the content of the buffering agent is comprised from 1% to 40% by weight over the total weight of the orodispersible powder composition.

In some embodiments, the orodispersible powder may comprise an antioxidant, which is usually selected from the group consisting of ascorbic acid, erythorbic acid, sodium ascorbate and mixtures thereof.

Generally, the antioxidant is usually present in an amount from 0.1% to 5% by weight over the total weight of the orodispersible powder composition.

### Manufacturing process

The process for preparing the orodispersible powder is a direct mixing of the components, which makes very attractive for industrial manufacturing. The process comprises generally sieving of the components through a 0.5 mm - 1 mm mesh sieve, and mixing in, for example, a V-shaped mixer, which is suitable for free-flowing powders. Mixing time is adjusted according to routine practice to obtain a homogeneous mixture, which is dosed in stick packs.

Conventional mixing procedures, well-known to the skilled in pharmaceutical technology, are disclosed in reference books in the field, for example, in the book Aulton's Pharmaceutics. The design and manufacture of medicines, M.E. Aulton and K.M.G. Taylor, editors, Churchill Livingstone Elsevier, Fourth Edition, 2013; or in the book Remington: The Science and Practice of Pharmacy, D. Limmer editor, Lippincott Williams & Wilkins, 2000.

### Stick pack

A stick pack comprising the orodispersible powder according to claim 1 also forms part of the invention.

A stick pack is a packaging form, usually having a laminate structure to give a specific barrier and sealing characteristics. It is used for a single dose administration. It is of common use containing pharmaceutical compositions or foods, e.g. sugar. Materials usually employed to manufacture stick packs are aluminium, paper, polyethylene, polyethylene terephthalate, propylene and combinations thereof.

The stick pack comprises a dose of a pharmacologically effective amount of the orodispersible powder composition, which comprises a triptan. The stick pack preferably comprises an amount of the orodispersible powder composition that comprises 2.5 mg naratriptan, 2.5 mg frovatriptan, 5 mg zolmitriptan, 10 mg rizatriptan, 12.5 mg almotriptan, 40 mg eletriptan or 100 mg sumatriptan, or a pharmaceutically acceptable salt thereof.

The stick pack usually comprises an amount of the orodispersible powder composition, which is comprised from 1 g to 10 g, preferably from 1.5 g to 5 g, more preferably from 1.9 g to 3 g, and yet more preferably about 2.2 g. Preferably it comprises 2.5 mg naratriptan, 2.5 mg frovatriptan, 5 mg zolmitriptan, 10 mg rizatriptan, 12.5 mg almotriptan, 40 mg eletriptan or 100 mg sumatriptan, or a pharmaceutically acceptable salt thereof. The new orodispersible powder formulation obtained by direct mixing of the components provides an excellent masking of the bitter taste of the triptans with an excellent acceptance of the formulation by the patient.

Orodispersible powders that are obtained by direct mixing for antimigraine active ingredients, such as triptans, and stick packs comprising the orodispersible powder composition obtained by direct mixing, are not commercially available.

Direct mixing of the components allows the direct exposure of the active ingredient in the oral cavity, increasing the dissolution rate of the active ingredient and its absorption in the oral cavity, favouring the fast effect of the medication, which is considered a very important attribute and valued by both patients and doctors.

In this invention the stick pack packaging form has been chosen because it is practical, robust, easy to transport and use individually, so it adapts well to a modern and active lifestyle. The patient can carry the stick pack always on top and, therefore, if a migraine attack occurs, the medication can be taken just after the onset of the migraine attack without the need to drink water, increasing the effectiveness of the triptans and decreasing early recurrence of moderate to severe headache. The orodispersible powder composition according to claim 1 has industrial application, since a direct mixing process is used as manufacturing process, which is widely used in the pharmaceutical industry, and, in addition, as shown in the examples, the formulation exhibits optimal rheological characteristics according to the European Pharmacopoeia current edition, and, therefore, it can be scale up at industrial level, so that it can be dosed on equipment with the stick pack packaging format.

As shown in the examples, the orodispersible powder composition according to claim 1 shows a high degree of acceptance by the patient for all triptans over a broad range of compositions.

### Use

The orodispersible powder composition according to claim 1 for use in the treatment of migraine, also forms part of the invention. In a preferred embodiment, the orodispersible powder composition according to claim 1 is for use in the acute treatment of the headache phase of migraine attacks, with or without aura, more preferably in adults.

In the following examples, different embodiments of the invention are provided for illustrative purposes that are understood to be non-limiting.

### Examples

### Examples 1-10: Orodispersible powders comprising sumatriptan

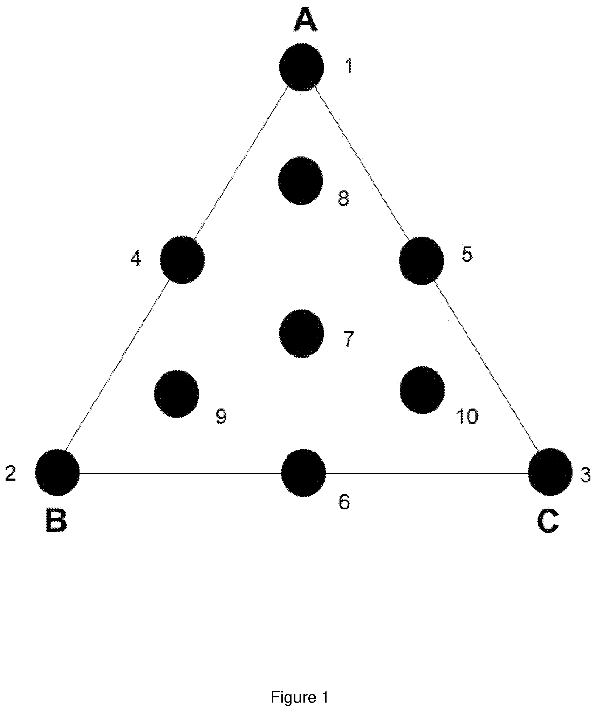

Orodispersible powders comprising sumatriptan as active ingredient were prepared according to a mixture design (see Figure 1). This methodology is well-known and disclosed, for example, in J. Cornell, Experiments with Mixtures, 3rd edition, John Wiley & Sons, 2002. The matrix of the design of experiments is shown in Table I:

**TABLE I**

| **Example** | **A** | **B** | **C** | **Citrate** | **Citric** | **Sucralose** | **Total** |
|---|---|---|---|---|---|---|---|
| 1* | 1 | 0 | 0 | 85 | 10 | 5 | 100 |
| 2* | 0 | 1 | 0 | 50 | 45 | 5 | 100 |
| 3* | 0 | 0 | 1 | 50 | 10 | 40 | 100 |
| 4* | 1/2 | 1/2 | 0 | 67.5 | 27.5 | 5 | 100 |
| 5* | 1/2 | 0 | 1/2 | 67.5 | 10 | 22.5 | 100 |
| 6* | 0 | 1/2 | 1/2 | 50 | 27.5 | 22.5 | 100 |
| 7 | 1/3 | 1/3 | 1/3 | 61.6 | 21.7 | 16.7 | 100 |
| 8 | 2/3 | 1/6 | 1/6 | 73.3 | 15.8 | 10.9 | 100 |
| 9 | 1/6 | 2/3 | 1/6 | 55.8 | 33.3 | 10.9 | 100 |
| 10 | 1/6 | 1/6 | 2/3 | 55.8 | 15.8 | 28.4 | 100 |

| | | | | | | | |
|---|---|---|---|---|---|---|---|
| * Outside the subject-matter of the claims, but suitable for understanding the invention | | | | | | | |

The specific compositions of these powders, expressed in mg, are shown in Table II:

**TABLE II**

| **Component** | **Ex. 1*** | **Ex. 2*** | **Ex. 3*** | **Ex. 4*** | **Ex. 5*** |
|---|---|---|---|---|---|
| | | | | | |
| Sumatriptan succinate | 140 | 140 | 140 | 140 | 140 |
| Sodium citrate anh. | 548.2 | 322.5 | 322.5 | 435.3 | 435.3 |
| Citric acid anh. | 64.5 | 290.2 | 64.5 | 177.4 | 64.5 |
| Sucralose | 32.3 | 32.3 | 258.0 | 32.3 | 145.2 |
| Mannitol | 1369 | 1369 | 1369 | 1369 | 1369 |
| Sodium stearyl fumarate | 11 | 11 | 11 | 11 | 11 |
| Colloidal silicon dioxide | 11 | 11 | 11 | 11 | 11 |
| Flavouring agent | 24 | 24 | 24 | 24 | 24 |

| **Component** | **Ex. 6*** | **Ex. 7** | **Ex. 8** | **Ex. 9** | **Ex. 10** |
|---|---|---|---|---|---|
| Sumatriptan succinate | 140 | 140 | 140 | 140 | 140 |
| Sodium citrate anh. | 322.5 | 397.3 | 472.8 | 359.9 | 359.9 |
| Citric acid anh. | 177.4 | 140.0 | 101.9 | 214.8 | 101.9 |
| Sucralose | 145.2 | 107.7 | 70.3 | 70.3 | 183.2 |
| Mannitol | 1369 | 1369 | 1369 | 1369 | 1369 |
| Sodium stearyl fumarate | 11 | 11 | 11 | 11 | 11 |
| Colloidal silicon dioxide | 11 | 11 | 11 | 11 | 11 |
| Flavouring agent | 24 | 24 | 24 | 24 | 24 |

| | | | | | |
|---|---|---|---|---|---|
| * Outside the subject-matter of the claims, but suitable for understanding the invention | | | | | |

In these examples the ratio active ingredient:ternary combination was 1 :6.45. The amount of 140 mg sumatriptan succinate corresponds to 100 mg sumatriptan, and the amount of the synergistic combination is 645 mg.

The process for preparing the orodispersible powders comprised the following steps:
1) Sieving the components through a 0.5 mm mesh sieve (except the citric acid anhydrous that is sieved through a 1 mm mesh sieve), and incorporate them into the V-shaped mixer in the following order: sodium citrate anhydrous, sucralose, flavouring agent, sumatriptan succinate, colloidal silicon dioxide, mannitol, and citric acid anhydrous.
2) Mixing about 15 minutes at about 20 rpm in the V-mixer.
3) Sieving the sodium stearyl fumarate through a 0.5 mm mesh sieve and incorporate it into the V-mixer.
4) Mix about 5 minutes at about 20 rpm in the V-mixer.
5) Dosing the product in stick packs (2200 mg per stick).

It was also confirmed that powders dissolve rapidly in the oral cavity, leaving no product residue in the mouth.

### Examples 11-20: Orodispersible powders comprising zolmitriptan

Orodispersible powders comprising zolmitriptan as active ingredient were prepared according to a mixture design (see Figure 1) and according to the matrix shown above in Table I.

The specific compositions of these powders, expressed in mg, are shown in Table III:

**TABLE III**

| **Component** | **Ex. 11*** | **Ex. 12*** | **Ex. 13*** | **Ex. 14*** | **Ex. 15*** |
|---|---|---|---|---|---|
| Zolmitriptan | 5 | 5 | 5 | 5 | 5 |
| Sodium citrate anh. | 336.6 | 198 | 198 | 267.3 | 267.3 |
| Citric acid anh. | 39.6 | 178.2 | 39.6 | 108.9 | 39.6 |
| Sucralose | 19.8 | 19.8 | 158.4 | 19.8 | 89.1 |
| Mannitol | 1759 | 1759 | 1759 | 1759 | 1759 |
| Sodium stearyl fumarate | 11 | 11 | 11 | 11 | 11 |
| Colloidal silicon dioxide | 11 | 11 | 11 | 11 | 11 |
| Flavouring agent | 18 | 18 | 18 | 18 | 18 |

| **Component** | **Ex. 16*** | **Ex. 17** | **Ex. 18** | **Ex. 19** | **Ex. 20** |
|---|---|---|---|---|---|
| Zolmitriptan | 5 | 5 | 5 | 5 | 5 |
| Sodium citrate anh. | 198 | 244 | 290.2 | 220.9 | 220.9 |
| Citric acid anh. | 108.9 | 85.9 | 62.6 | 131.9 | 62.6 |
| Sucralose | 89.1 | 66.1 | 43.2 | 43.2 | 112.5 |
| Mannitol | 1759 | 1759 | 1759 | 1759 | 1759 |
| Sodium stearyl fumarate | 11 | 11 | 11 | 11 | 11 |
| Colloidal silicon dioxide | 11 | 11 | 11 | 11 | 11 |
| Flavouring agent | 18 | 18 | 18 | 18 | 18 |

| | | | | | |
|---|---|---|---|---|---|
| * Outside the subject-matter of the claims, but suitable for understanding the invention | | | | | |

In these examples the ratio active ingredient:ternary combination was 1:79.2, i.e. 5 mg of zolmitriptan and 396 mg of the synergistic combination.

The process for preparing the orodispersible powders is analogous to the process disclosed above in Examples 1 to 10.

It was also confirmed that powders dissolve rapidly in the oral cavity, leaving no product residue in the mouth.

### Example 21: Orodispersible powder comprising almotriptan

Following an analogous process disclosed above in Examples 1 to 10, an orodispersible powder comprising almotriptan malate as active ingredient was prepared according to the composition, expressed in mg, of Table IV:

**TABLE IV**

| **Component** | **Ex. 21** |
|---|---|
| Almotriptan malate | 17.5 |
| Sodium citrate anh. | 300 |
| Citric acid anh. | 72 |
| Sucralose | 24 |
| Mannitol | 1746.5 |
| Sodium stearyl fumarate | 11 |
| Colloidal silicon dioxide | 11 |
| Flavouring agent | 18 |

The amount of 17.5 mg of almotriptan malate corresponds to 12.5 mg of almotriptan. In this example the ratio active ingredient:ternary combination was 1 :31.68, i.e. 12.5 mg of almotriptan and 396 mg of the synergistic combination.

It was also confirmed that the powder dissolves rapidly in the oral cavity, leaving no product residue in the mouth.

### Example 22: Orodispersible powder comprising rizatriptan

Following an analogous process disclosed above in Examples 1 to 10, an orodispersible powder comprising rizatriptan benzoate as active ingredient was prepared according to the composition, expressed in mg, of Table V:

**TABLE V**

| **Component** | **Ex. 22** |
|---|---|
| Rizatriptan benzoate | 14.53 |
| Sodium citrate anh. | 300 |
| Citric acid anh. | 72 |
| Sucralose | 24 |
| Mannitol | 1749.47 |
| Sodium stearyl fumarate | 11 |
| Colloidal silicon dioxide | 11 |
| Flavouring agent | 18 |

The amount of 14.53 mg of rizatriptan benzoate corresponds to 10 mg of rizatriptan. In this example the ratio active ingredient:ternary combination was 1:39.6, i.e. 10 mg of rizatriptan and 396 mg of the synergistic combination.

It was also confirmed that the powder dissolves rapidly in the oral cavity, leaving no product residue in the mouth.

### Example 23: Orodispersible powder comprising eletriptan

Following an analogous process disclosed above in Examples 1 to 10, an orodispersible powder comprising eletriptan hydrobromide as active ingredient was prepared according to the composition, expressed in mg, of Table VI:

**TABLE VI**

| **Component** | **Ex. 23** |
|---|---|
| Eletriptan hydrobromide | 48.48 |
| Sodium citrate anh. | 400 |
| Citric acid anh. | 130 |
| Sucralose | 125 |
| Mannitol | 1450.52 |
| Sodium stearyl fumarate | 11 |
| Colloidal silicon dioxide | 11 |
| Flavouring agent | 24 |

The amount of 48.48 mg of eletriptan hydrobromide corresponds to 40 mg of eletriptan. In this example the ratio active ingredient:ternary combination was 1 :16.37, i.e. 40 mg of eletriptan and 655 mg of the synergistic combination.

It was also confirmed that the powder dissolves rapidly in the oral cavity, leaving no product residue in the mouth.

### Example 24: Orodispersible powder comprising zolmitriptan

Following an analogous process disclosed above in Examples 1 to 10, an orodispersible powder comprising zolmitriptan as active ingredient was prepared according to the composition, expressed in mg, of Table VII:

**TABLE VII**

| **Component** | **Ex. 24** |
|---|---|
| Zolmitriptan | 5 |
| Sodium citrate anh. | 300 |
| Citric acid anh. | 72 |
| Sucralose | 24 |
| Mannitol | 1759 |
| Sodium stearyl fumarate | 11 |
| Colloidal silicon dioxide | 11 |
| Flavouring agent | 18 |

In this example the ratio active ingredient:ternary combination was 1:79.2, i.e. 5 mg of zolmitriptan and 396 mg of the synergistic combination.

It was also confirmed that the powder dissolves rapidly in the oral cavity, leaving no product residue in the mouth.

### Example 25: Orodispersible powder comprising sumatriptan

Following an analogous process disclosed above in Examples 1 to 10, an orodispersible powder comprising sumatriptan succinate as active ingredient was prepared according to the composition, expressed in mg, of Table VIII:

**TABLE VIII**

| **Component** | **Ex. 25** |
|---|---|
| Sumatriptan succinate | 140 |
| Sodium citrate anh. | 400 |
| Citric acid anh. | 130 |
| Sucralose | 115 |
| Mannitol | 1369 |
| Sodium stearyl fumarate | 11 |
| Colloidal silicon dioxide | 11 |
| Flavouring agent | 24 |

In this example the ratio active ingredient:ternary combination was 1 :6.45. The amount of 140 mg sumatriptan succinate corresponds to 100 mg sumatriptan, and the amount of the synergistic combination is 645 mg.

It was also confirmed that the powder dissolves rapidly in the oral cavity, leaving no product residue in the mouth.

### Example 26: Testing of orodispersible powders comprising triptans

### a) Organoleptic testing

Organoleptic tests of the orodispersible powder formulations dosed in stick packs, were carried out in 5 healthy volunteers.

The methodology for evaluating the acceptance of the formulations by the patient has consisted in establishing a score from 1 to 5 for the taste of the formulation (acid, sweet or salty), where 1 corresponds to an unacceptable taste, and 5 corresponds to an excellent taste, and another score from 1 to 5 to assess the bitterness of the formulation, wherein 1 corresponds to an unacceptable bitterness, and 5 corresponds to an excellent capacity for masking the bitterness of the active ingredient. The multiplication of the taste value of the formulation by the value referring to the capacity of masking the bitterness of the active ingredient, gives rise to a value that is the degree of acceptance of the formulation by the patient.

The results obtained indicate that the formulations mask the bitter taste of the triptans, with an excellent acceptance of the formulation by the patient, and without the need to drink water after the administration of the orodispersible formulation. Table IX shows the results of several orodispersible powder formulations suitable for understanding the invention:

**TABLE IX**

| **Example** | **Bitterness** | **Taste** | **Degree of Acceptance** |
|---|---|---|---|
| 1 * | 1 | 3 | 3 |
| 2* | 3 | 1 | 3 |
| 3* | 2 | 4 | 8 |
| 4* | 4 | 3 | 12 |
| 5* | 3 | 4 | 12 |
| 6* | 4 | 3 | 12 |
| 7 | 5 | 5 | 25 |
| 8 | 3 | 5 | 15 |
| 9 | 5 | 3 | 15 |
| 10 | 5 | 4 | 20 |
| 11* | 5 | 1 | 5 |
| 12* | 5 | 1 | 5 |
| 13* | 4 | 2 | 8 |
| 14* | 5 | 3 | 15 |
| 15* | 4 | 3 | 12 |
| 16* | 5 | 3 | 15 |
| 17 | 5 | 5 | 25 |
| 18 | 5 | 4 | 20 |
| 19 | 5 | 4 | 20 |
| 20 | 5 | 4 | 20 |
| 21 | 5 | 5 | 25 |
| 22 | 5 | 5 | 25 |
| 23 | 5 | 5 | 25 |
| 24 | 5 | 5 | 25 |
| 25 | 5 | 5 | 25 |

| | | | |
|---|---|---|---|
| * Outside the subject-matter of the claims, but suitable for understanding the invention | | | |

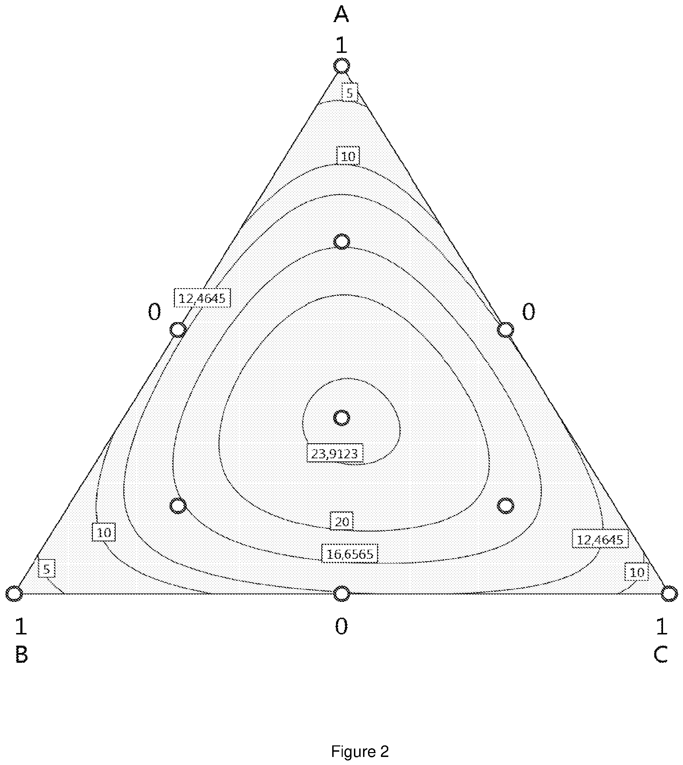

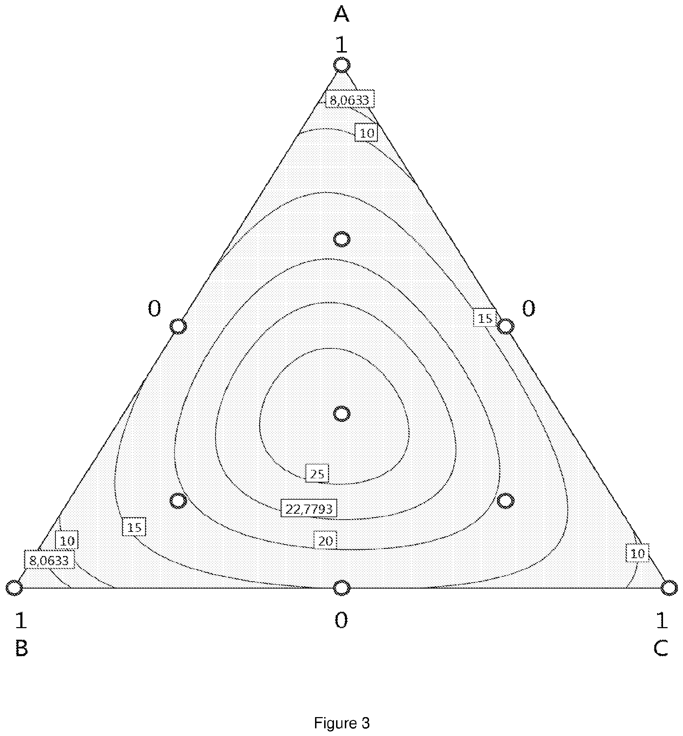

Figures 2 and 3 represent the isolines of the degree of acceptance of the orodispersible powders, prepared according to a mixture design, and comprising sumatriptan and zolmitriptan respectively, as function of the composition of the synergistic ternary combination.

It can be observed that the ternary combinations provide a synergistic effect regarding acceptance of the formulation by the patient. Further, the special cubic model described in a statistically significant way the acceptance behaviour of the mixtures.

The dissolution rates in the buccal cavity of the orodispersible powders dosed in stick packs, prepared according to Examples 21 to 25, were carried out in 5 healthy volunteers. The results indicated that the formulations show a very rapid dissolution (about 10 seconds) in the buccal cavity, without leaving any residue of product in the mouth, and they were well accepted by the panel of testers.

### b) Physical parameters

Bulk density and tapped density were determined according to the methodology of the European Pharmacopoeia current edition, and the compressibility index and the Hausner ratio were calculated for several orodispersible powders according to the invention.

Table X shows bulk density (g/ml), tapped density (g/ml), pH (1% w/v in purified water) and moisture (%):

**TABLE X**

| **Parameter** | **Ex. 21** | **Ex. 22** | **Ex. 23** | **Ex. 24** | **Ex. 25** |
|---|---|---|---|---|---|
| Bulk density | 0.71 | 0.72 | 0.75 | 0.72 | 0.74 |
| Tapped density | 0.80 | 0.79 | 0.83 | 0.78 | 0.84 |
| pH | 3.6 | 4.3 | 3.9 | 4.3 | 4.1 |
| Moisture | 1.6 | 1.1 | 1.9 | 1.6 | 2.3 |

Moisture values are comprised between 1% and 3%, which is an optimal range for direct mixtures.

The following galenic parameters were determined in order to assess the flowability of the formulations: angle of repose (degrees), compressibility index (%) and Hausner ratio. The methodology of the European Pharmacopoeia current edition was used, as shown in Table XI:

**TABLE XI**

| **Compressibility index** | **Hausner ratio** | **Angle of repose** | **Assessment** |
|---|---|---|---|
| 1-10 | 1.00-1.11 | 25-30 | Excellent |
| 11-15 | 1.12-1.18 | 31-35 | Good |
| 16-20 | 1.19-1.25 | 36-40 | Fair |
| 21-25 | 1.26-1.34 | 41-45 | Passable |
| 26-31 | 1.35-1.45 | 46-55 | Poor |
| 32-37 | 1.46-1.59 | 56-65 | Very poor |
| >38 | >1.60 | >66 | Very, very poor |

Table XII shows the results for several orodispersible powders according to the invention:

**TABLE XII**

| **Parameter** | **Ex. 21** | **Ex. 22** | **Ex. 23** | **Ex. 24** | **Ex. 25** |
|---|---|---|---|---|---|
| Compressibility index | 11.3 | 8.9 | 9.6 | 7.7 | 11.9 |
| Hausner ratio | 1.13 | 1.10 | 1.11 | 1.08 | 1.14 |
| Angle of repose | 29 | 29 | 28 | 29 | 25 |

The results indicate that the formulations have optimal flow properties, which were maintained even after 6 months storage at room temperature.

## Claims

1. An orodispersible powder composition, **characterized in that** it comprises:
a) a pharmacologically effective amount of a triptan or a pharmaceutically acceptable salt thereof,
b) a ternary combination consisting of sodium citrate, citric acid and sucralose, and
c) one or more further excipients,
wherein the ratio a):b) is comprised from 1:2 to 1:90,
wherein the content of sodium citrate is comprised from 55% to 75% by weight over the total weight of the ternary combination,
wherein the content of citric acid is comprised from 15% to 35% by weight over the total weight of the ternary combination, and
wherein the content of sucralose is comprised from 10% to 30% by weight over the total weight of the ternary combination.

2. The orodispersible powder composition according to claim 1, **characterized in that** the triptan is selected from the group of sumatriptan, zolmitriptan, eletriptan, rizatriptan, almotriptan, frovatriptan, naratriptan, donitriptan, avitriptan, LY-334370, L-703,664 or GR 46611.

3. The orodispersible powder composition according to any one of claims 1 to 2, **characterized in that** the ratio triptan: ternary combination is selected from the group comprising: 1:2, 1:3, 1:4, 1:5, 1:6.5, 1:10, 1:15, 1:16, 1:20, 1:25, 1:30, 1:32, 1:35, 1:40, 1:45, 1:50, 1:55, 1:60, 1:65, 1:70, 1:75, 1:79, 1:80, 1:85, and 1:90.

4. The orodispersible powder composition according to any one of claims 1 to 3, **characterized in that** the content of sodium citrate is comprised from 60% to 70% by weight over the total weight of the ternary combination.

5. The orodispersible powder composition according to any one of claims 1 to 4, **characterized in that** the content of citric acid is comprised from 20% to 30% by weight over the total weight of the ternary combination.

6. The orodispersible powder composition according to any one of claims 1 to 5, **characterized in that** the content of sucralose is comprised from 15% to 25% by weight over the total weight of the ternary combination.

7. The orodispersible powder composition according to any one of claims 1 to 6, **characterized in that** one or more further excipients are selected from diluents, lubricants, glidants, flavouring agents, sweetening agents, acidulants, alkalizing agents, buffering agents, antioxidants and mixtures thereof.

8. The orodispersible powder composition according to claim 7, **characterized in that** the diluent is selected from mannitol, sorbitol, xylitol, isomalt, erythritol, cellulose powdered, microcrystalline cellulose, silified microcrystalline cellulose, starch, calcium phosphate, calcium lactate, calcium carbonate, magnesium carbonate, magnesium oxide and mixtures thereof.

9. The orodispersible powder composition according to claim 7, **characterized in that** the lubricant is selected from the group consisting of talc, sodium benzoate, sodium stearyl fumarate, sodium lauryl sulfate, calcium stearate, magnesium stearate, zinc stearate, glyceryl behenate, stearic acid, glyceryl monostearate, poloxamer, polyethylene glycol and mixtures thereof.

10. The orodispersible powder composition according to claim 7, **characterized in that** the glidant is selected from the group consisting of colloidal silicon dioxide, calcium phosphate tribasic, hydrophobic colloidal silica, magnesium silicate, magnesium trisilicate, silicon dioxide, talc and mixtures thereof.

11. The orodispersible powder composition according to claim 7, **characterized in that** the flavouring agent is selected from orange, banana, strawberry, cherry, wild cherry, lemon, cardamom, anise, peppermint, menthol, vanillin and ethyl vanillin and mixtures thereof.

12. A stick pack comprising the orodispersible powder according to any one of claims 1 to 11.

13. The stick pack according to claim 12, **characterized in that** it comprises an amount of the orodispersible powder composition, which is comprised from 1 g to 10 g, preferably from 1.5 g to 5 g, more preferably from 1.9 g to 3 g, and yet more preferably about 2.2 g.

14. The orodispersible powder composition according to any one of claims 1 to 11 for use in the treatment of migraine.

15. The orodispersible powder composition according to claim 14 for use in the acute treatment of the headache phase of migraine attacks, with or without aura, more preferably in adults.

## Patentansprüche

1. Schmelzpulverzusammensetzung, **dadurch gekennzeichnet, dass** sie Folgendes umfasst:
a) eine pharmakologisch wirksame Menge eines Triptans oder eines pharmazeutisch verträglichen Salzes davon,
b) eine ternäre Kombination bestehend aus Natriumcitrat, Zitronensäure und Sucralose, und
c) einen oder mehrere weitere Hilfsstoffe,
wobei das Verhältnis a):b) zwischen 1:2 und 1:90 umfasst,
wobei der Gehalt an Natriumcitrat zwischen 55 und 75 Gew.-% umfasst, bezogen auf das Gesamtgewicht der ternären Kombination,
wobei der Gehalt an Zitronensäure zwischen 15 und 35 Gew.-% umfasst, bezogen auf das Gesamtgewicht der ternären Kombination, und
wobei der Gehalt an Sucralose zwischen 10 und 30 Gew.-% umfasst, bezogen auf das Gesamtgewicht der ternären Kombination.

2. Schmelzpulverzusammensetzung nach Anspruch 1, **dadurch gekennzeichnet, dass** das Triptan aus der Gruppe Sumatriptan, Zolmitriptan, Eletriptan, Rizatriptan, Almotriptan, Frovatriptan, Naratriptan, Donitriptan, Avitriptan, LY-334370, L-703,664 oder GR 46611 ausgewählt ist.

3. Schmelzpulverzusammensetzung nach einem der Ansprüche 1 bis 2, **dadurch gekennzeichnet, dass** das Verhältnis Triptan: ternäre Kombination aus der Gruppe ausgewählt ist, die Folgendes umfasst: 1:2, 1:3, 1:4, 1:5, 1:6,5, 1:10, 1:15, 1:16, 1:20, 1:25, 1:30, 1:32, 1:35, 1:40, 1:45, 1:50, 1:55, 1:60, 1:65, 1:70, 1:75, 1:79, 1:80, 1:85 und 1:90.

4. Schmelzpulverzusammensetzung nach einem der Ansprüche 1 bis 3, **dadurch gekennzeichnet, dass** der Gehalt an Natriumcitrat zwischen 60 und 70 Gew.-% umfasst, bezogen auf das Gesamtgewicht der ternären Kombination.

5. Schmelzpulverzusammensetzung nach einem der Ansprüche 1 bis 4, **dadurch gekennzeichnet, dass** der Gehalt an Zitronensäure zwischen 20 und 30 Gew.-% umfasst, bezogen auf das Gesamtgewicht der ternären Kombination.

6. Schmelzpulverzusammensetzung nach einem der Ansprüche 1 bis 5, **dadurch gekennzeichnet, dass** der Gehalt an Sucralose zwischen 15 und 25 Gew.-% umfasst, bezogen auf das Gesamtgewicht der ternären Kombination.

7. Schmelzpulverzusammensetzung nach einem der Ansprüche 1 bis 6, **dadurch gekennzeichnet, dass** ein oder mehrere weitere Hilfsstoffe aus Verdünnungsmitteln, Schmiermitteln, Gleitmitteln, Aromastoffen, Süßungsmitteln, Säuerungsmitteln, Alkalisierungsmitteln, Puffermitteln, Antioxidantien und Mischungen davon ausgewählt sind.

8. Schmelzpulverzusammensetzung nach Anspruch 7, **dadurch gekennzeichnet, dass** das Verdünnungsmittel aus Mannit, Sorbit, Xylit, Isomalt, Erythrit, Cellulosepulver, mikrokristalliner Cellulose, silifizierter mikrokristalliner Cellulose, Stärke, Calciumphosphat, Calciumlactat, Calciumcarbonat, Magnesiumcarbonat, Magnesiumoxid und Mischungen davon ausgewählt ist.

9. Schmelzpulverzusammensetzung nach Anspruch 7, **dadurch gekennzeichnet, dass** das Schmiermittel aus der Gruppe bestehend aus Talk, Natriumbenzoat, Natriumstearylfumarat, Natriumlaurylsulfat, Calciumstearat, Magnesiumstearat, Zinkstearat, Glycerylbehenat, Stearinsäure, Glycerylmonostearat, Poloxamer, Polyethylenglykol und Mischungen davon ausgewählt ist.

10. Schmelzpulverzusammensetzung nach Anspruch 7, **dadurch gekennzeichnet, dass** das Gleitmittel aus der Gruppe bestehend aus kolloidalem Siliciumdioxid, tribasischem Calciumphosphat, hydrophobem kolloidalem Siliciumdioxid, Magnesiumsilicat, Magnesiumtrisilicat, Siliciumdioxid, Talk und Mischungen davon ausgewählt ist.

11. Schmelzpulverzusammensetzung nach Anspruch 7, **dadurch gekennzeichnet, dass** der Aromastoff aus Orange, Banane, Erdbeere, Kirsche, Wildkirsche, Zitrone, Kardamom, Anis, Pfefferminze, Menthol, Vanillin und Ethylvanillin und Mischungen davon ausgewählt ist.

12. Stickpack, umfassend das Schmelzpulver nach einem der Ansprüche 1 bis 11.

13. Stickpack nach Anspruch 12, **dadurch gekennzeichnet, dass** es eine Menge der Schmelzpulverzusammensetzung umfasst, die zwischen 1 g und 10 g, vorzugsweise zwischen 1,5 g und 5 g, noch bevorzugter zwischen 1,9 g und 3 g und sogar noch bevorzugter etwa 2,2 g umfasst.

14. Schmelzpulverzusammensetzung nach einem der Ansprüche 1 bis 11 zur Verwendung bei der Behandlung von Migräne.

15. Schmelzpulverzusammensetzung nach Anspruch 14 zur Verwendung bei der akuten Behandlung der Kopfschmerzphase von Migräneanfällen mit oder ohne Aura, besonders bevorzugt bei Erwachsenen.

## Revendications

1. Composition de poudre orodispersible, **caractérisée en ce qu'**elle comprend :
a) une quantité pharmacologiquement efficace d'un triptan ou d'un sel pharmaceutiquement acceptable de celui-ci,
b) une combinaison ternaire constituée de citrate de sodium, d'acide citrique et de sucralose et
c) un ou plusieurs autres excipients,
dans laquelle le rapport a):b) est compris entre 1:2 et 1:90,
dans laquelle la teneur en citrate de sodium est comprise entre 55 % et 75 % en poids par rapport au poids total de la combinaison ternaire,
dans laquelle la teneur en acide citrique est comprise entre 15 % et 35 % en poids par rapport au poids total de la combinaison ternaire et
dans laquelle la teneur en sucralose est comprise entre 10 % et 30 % en poids par rapport au poids total de la combinaison ternaire.

2. Composition de poudre orodispersible selon la revendication 1, **caractérisée en ce que** le triptan est choisi dans le groupe du sumatriptan, du zolmitriptan, de l'élétriptan, du rizatriptan, de l'almotriptan, du frovatriptan, du naratriptan, du donitriptan, de l'avitriptan, de LY-334370, de L-703 664 ou de GR 46611.

3. Composition de poudre orodispersible selon l'une quelconque des revendications 1 à 2, **caractérisée en ce que** le rapport triptan combinaison ternaire est choisi dans le groupe comprenant : 1:2, 1:3, 1:4, 1:5, 1:6,5, 1:10, 1:15, 1:16, 1:20, 1:25, 1:30, 1:32, 1:35, 1:40, 1:45, 1:50, 1:55, 1:60, 1:65, 1:70, 1:75, 1:79, 1:80, 1:85 et 1:90.

4. Composition de poudre orodispersible selon l'une quelconque des revendications 1 à 3, **caractérisée en ce que** la teneur en citrate de sodium est comprise entre 60 % et 70 % en poids par rapport au poids total de la combinaison ternaire.

5. Composition de poudre orodispersible selon l'une quelconque des revendications 1 à 4, **caractérisée en ce que** la teneur en acide citrique est comprise entre 20 % et 30 % en poids par rapport au poids total de la combinaison ternaire.

6. Composition de poudre orodispersible selon l'une quelconque des revendications 1 à 5, **caractérisée en ce que** la teneur en sucralose est comprise entre 15 % et 25 % en poids par rapport au poids total de la combinaison ternaire.

7. Composition de poudre orodispersible selon l'une quelconque des revendications 1 à 6, **caractérisée en ce qu'**un ou plusieurs autres excipients sont choisis parmi des diluants, des lubrifiants, des agents de glissement, des agents aromatisants, des agents édulcorants, des acidulants, des agents alcalinisants, des agents tampons, des antioxydants et des mélanges de ceux-ci.

8. Composition de poudre orodispersible selon la revendication 7, **caractérisée en ce que** le diluant est choisi parmi le mannitol, le sorbitol, le xylitol, l'isomalt, l'érythritol, la cellulose en poudre, la cellulose microcristalline, la cellulose microcristalline silicifiée, l'amidon, le phosphate de calcium, le lactate de calcium, le carbonate de calcium, le carbonate de magnésium, l'oxyde de magnésium et des mélanges de ceux-ci.

9. Composition de poudre orodispersible selon la revendication 7, **caractérisée en ce que** le lubrifiant est choisi dans le groupe constitué par le talc, le benzoate de sodium, le stéarylfumarate de sodium, le laurylsulfate de sodium, le stéarate de calcium, le stéarate de magnésium, le stéarate de zinc, le béhénate de glycéryle, l'acide stéarique, le monostéarate de glycéryle, un poloxamère, le polyéthylèneglycol et les mélanges de ceux-ci.

10. Composition de poudre orodispersible selon la revendication 7, **caractérisée en ce que** l'agent de glissement est choisi dans le groupe constitué par le dioxyde de silicium colloïdal, le phosphate tricalcique, la silice colloïdale hydrophobe, le silicate de magnésium, le trisilicate de magnésium, le dioxyde de silicium, le talc et les mélanges de ceux-ci.

11. Composition de poudre orodispersible selon la revendication 7, **caractérisée en ce que** l'agent aromatisant est choisi parmi l'orange, la banane, la fraise, la cerise, la cerise douce, le citron, la cardamome, l'anis, la menthe poivrée, le menthol, la vanilline et l'éthylvanilline et des mélanges de ceux-ci.

12. Emballage sous forme de stick comprenant la poudre orodispersible selon l'une quelconque des revendications 1 à 11.

13. Emballage sous forme de stick selon la revendication 12, **caractérisé en ce qu'**il comprend une quantité de la composition de poudre orodispersible, qui est comprise entre 1g et 10 g, de préférence entre 1,5 g et 5 g, plus préférablement entre 1,9 g et 3 g et encore plus préférablement qui est d'environ 2,2 g.

14. Composition de poudre orodispersible selon l'une quelconque des revendications 1 à 11 destinée à être utilisée dans le traitement de la migraine.

15. Composition de poudre orodispersible selon la revendication 14 destinée à être utilisée dans le traitement aigu de la phase de céphalée de crises de migraine, avec ou sans aura, plus préférablement chez les adultes.
